# EUROPEAN PATENT APPLICATION

(11) **EP 4 763 203 A1**
(43) Date of publication of application: **24.06.2026**
(21) Application number: 25226545.9
(22) Date of filing: 22.12.2025
(51) Int. Cl.: A61K 31/216, A61P 35/00

(54) **N-(1-ADAMANTYL)BENZAMIDE DERIVATIVES FOR THE TREATMENT OF GASTRIC CARCINOMA**

(30) Priority: 23.12.2024 IT 202400029760
(71) Applicant: Università degli Studi di Bari "Aldo Moro", 70121 Bari (BA) (IT); Ente Ospedaliero Specializzato in Gastroenterologia "Saverio de Bellis" - IRCCS, 70013 Castellana Grotte (BA) (IT)
(72) Inventor: ABATE, Carmen, I-70126 BARI (IT); COLABUFO, Nicola Antonio, I-70019 TRIGGIANO (Bari) (IT); CONTINO, Marialessandra, I-70125 BARI (IT); D'ALESSANDRO, Rosalba, I-70013 CASTELLANA GROTTE (Bari) (IT); GIANNELLI, Gianluigi, I-70013 CASTELLANA GROTTE (Bari) (IT); LOTESORIERE, Claudio, I-70013 CASTELLANA GROTTE (Bari) (IT); SCHIRIZZI, Annalisa, I-70013 CASTELLANA GROTTE (Bari) (IT); STEFANACHI, Angela, I-70126 BARI (IT)
(74) Representative: Comoglio, Elena

(57) **Abstract**

The invention refers to N-(1-adamantyl)benzamide derivatives which are dual agents, cannabinoid type 2 receptor (CB2R) agonists, and fatty acid amide hydrolase (FAAH) inhibitors, for use in the therapeutic treatment of gastric carcinoma, optionally in combination with one or more chemotherapeutic agents.

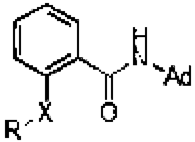

## Description

### Technical Field

The present invention relates to the field of oncology. More specifically, the invention refers to multitarget ligands to inhibit tumor growth, overcome chemoresistance, and improve the effectiveness of conventional chemotherapy agents in the treatment of gastric carcinoma.

### Background art

Gastric carcinoma is the third highest-mortality-rate malignancy in the world, affecting more than one million people each year. It is a very heterogeneous tumor, both histopathologically and molecularly. In its early stages, the disease is often asymptomatic, leading to late diagnosis, which decreases the chances of the tumor being resectable. In fact, only about 20% of patients have a disease that can be resected at diagnosis. Unfortunately, even in the case of curative resection, the prognosis remains poor due to the high recurrence rate. The 5-year patient survival rate is in fact less than 10%.

These characteristics often limit the therapy to treatment with chemotherapeutic agents which, however, have the disadvantage of acting indiscriminately on all cells of the body, resulting in high cytotoxicity and side effects.

Paclitaxel, a member of the taxane family of which docetaxel and irinotecan are also members, is one of the chemotherapeutic drugs conventionally used in the second-line treatment of gastric carcinoma, either alone or in combination with Ramucirumab. Other chemotherapeutics conventionally used, alone or in combination, belong to the families of platinum derivatives (cisplatin, oxaliplatin) and fluoropyrimidines (fluorouracil (5-FU), capecitabine, tegafur (S-1).

However, the effectiveness of conventional chemotherapeutics in the treatment of gastric carcinoma is severely limited due to the chemoresistance phenomenon, i.e., the development of resistance to the effects of drugs by tumor cells.

The paper in J Med Chem. 2023;66(1):235-250. doi:10.1021/acs.jmedchem.2c01084 describes the development of a number of multitarget agents with an N-(1-adamantyl)benzamide structure, such as dual modulators, cannabinoid type 2 receptor (CB2R) activators, and fatty acid amide hydrolase (FAAH) inhibitors. The study concludes that some of the synthesized N-(1-adamantyl)benzamide derivatives, particularly compounds designated as 13, 26, and 27, are effective CB2R and FAAH modulators, which could offer a promising approach for the treatment of inflammatory diseases and conditions. Although the paper mentions cancer as a disease characterized by inflammatory states, no *in vitro* or *in vivo* studies have been described in which the effectiveness of N-(1-adamantyl)benzamide derivatives has been investigated in the treatment of cancer, especially gastric cancer.

### Summary of the invention

In the light of the state of the art, therefore, there is still a need to develop new therapeutic approaches for the treatment of gastric carcinoma, which do not suffer from the limitations of conventional chemotherapeutics, in particular with regard to the development of the chemoresistance phenomenon and the cytotoxicity characteristics.

Therefore, one object of the present invention is to provide a pharmaceutical active ingredient which is effective in the therapeutic treatment of gastric carcinoma, the administration of which does not lead to the development of chemoresistance.

A further object of the present invention is to provide a pharmaceutical active ingredient which is effective in the therapeutic treatment of gastric carcinoma and exhibits low cytotoxicity.

A still further object of the present invention is to provide a pharmaceutical active ingredient which is effective in the therapeutic treatment of gastric carcinoma and represents a valuable support to conventional therapies, improving their effectiveness in patients who have developed chemoresistance.

These and other objects have been achieved by the present inventors, who have found that some N-(1-adamantyl)benzamide derivatives, as defined in the attached claim 1, have a targeted action on cell proliferation and apoptosis processes, reducing tumor growth in gastric carcinoma and also showing low cytotoxicity. Even more importantly, and quite surprisingly, the aforementioned compounds also showed a synergistic effect with paclitaxel, reactivating the inhibitory effect of the chemotherapeutic drug even in resistance settings. The results obtained by the present inventors demonstrate that the aforementioned compounds are not only effective in the therapeutic treatment of gastric carcinoma as such, but also represent a valuable support to conventional therapies by improving their effectiveness and overcoming the chemoresistance problem.

### Detailed description

The present invention therefore relates to a compound of formula (I) as defined in the attached claim 1 for use in the therapeutic treatment of gastric carcinoma.

Formula (I) is shown below: 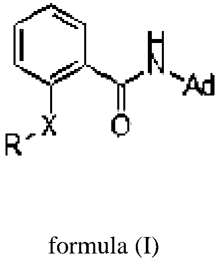 wherein:
X is selected from NH and O,
Ad is adamantyl,
R is selected from the group consisting of linear C1-C5 alkyl, phenyl, benzyl, and methyl cyclohexyl.

All attached claims form an integral part of the present specification.

The scope of the invention also includes the use of a compound of formula (I) in the therapeutic treatment of gastric carcinoma in combination with one or more chemotherapeutic agents, preferably paclitaxel optionally in combination with Ramucirumab.

In a preferred embodiment of the invention, the compound of formula (I) and the one or more chemotherapeutic agents are combined in a pharmaceutical composition formulated, for example, for oral or intravenous administration, for use in the therapeutic treatment of gastric carcinoma. Excipients and/or carriers suitable for the manufacture of a pharmaceutical composition depend largely on the administration mode selected, and in any case the selection and use thereof fall within the skills of the person skilled in the art.

In another preferred embodiment of the invention, the compound of formula (I) and the one or more chemotherapeutic agents are provided in the form of a combined preparation, for simultaneous, separate, or sequential use in the therapeutic treatment of gastric carcinoma. In the combined preparation of the invention, the compound of formula (I) is preferably formulated for oral or intravenous administration USING pharmaceutically acceptable carriers and/or excipients, the selection and use of which fall within the skills of the person skilled in the art.

In all embodiments of invention, the gastric carcinoma is preferably advanced gastric carcinoma.

The dose of the compound of formula (I) to be administered to the patient suffering from gastric carcinoma depends on the patient's characteristics, the stage of the disease, and the characteristics of the disease. Based on the minimum effective dose tested in mice by the inventors (10 mg/kg, see experimental part) and applying the Human Equivalent Dose (HED) equation, it is calculated that, for a human weighing approximately 60 kg, a therapeutically effective dose is reasonably between 1 mg and 20 mg.

In embodiments of the invention involving the combined use of a compound of formula (I) and one or more chemotherapeutic agents, the use of the chemotherapeutic agent paclitaxel, optionally in combination with Ramucirumab, is preferred.

Preferred compounds of formula (I) for use in the invention are the following:

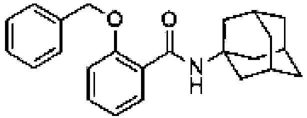

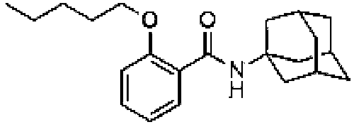

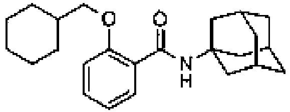

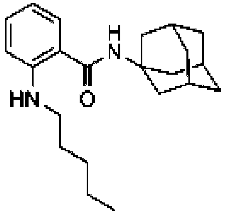

These compounds are capable of activating the CB2 receptor, binding to it with high affinity and good selectivity compared to CB1R (i.e., the endocannabinoid receptor responsible for psychotropic effects), and of inhibiting the activity of the FAAH enzyme, preventing the degradation of anandamide and other lipid derivatives responsible for the antitumor activity.

These results are illustrated in the table below which refers to the scientific papers Eur J Med Chem.2023;248:115109.doi:10.1016/j.ejmech.2023.115109 and J Med Chem. 2023;66(1):235-250. doi:10.1021/acs.jmedchem.2c01084.

**Table**

| Name | Structure | Affinity for CB1R *K*ᵢ, nM or % @ µM. | Affinity for CB2R *K*ᵢ, nM | FAAH IC₅₀ µM | CB2R activity EC₅₀, nM |
|---|---|---|---|---|---|
| FI5 CC48 (13) JMC2023 | 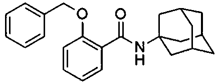 | 241.3 | 14.8 | 4.0 | Agonist 123.6 |
| FI8 (26) JMC2023 | 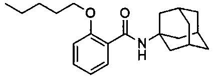 | 152.9 | 10.8 | 6.2 | Agonist 86.9 |
| Fi9 (27) JMC2023 | 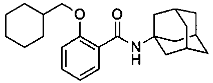 | 67.6 | 20.1 | 3.4 | Agonist 283.3 |
| ASF 151 (4) EJMC2023 | 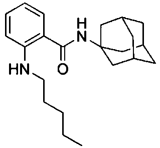 | 31% | 55.7 | 6.4 | Agonist 560 |

Among the specific compounds indicated above the most preferred is: 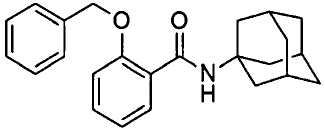 (for brevity referred to as "CC48").

This compound was tested *in vitro* and *in vivo* for its antitumor activity on gastric carcinoma cell lines.

In light of the results obtained with CC48 (described below) and considering that the preferred compounds listed above share with CC48 the basic chemical structure, CB2R agonist activity, selectivity as compared with CB1R and FAAH enzyme inhibitory activity, it is plausible that they also have biological activities similar to those of CC48.

*In vitro* studies performed by the present inventors with CC48 were carried out on both the commercial paclitaxel-sensitive human gastric carcinoma cell line named HGC27-S and a paclitaxel-resistant human gastric carcinoma cell line named HGC27-R. The HGC27-R cell line was created from the HGC27-S cell line by treating the cells with increasing concentrations of paclitaxel (PTX) starting at a concentration of 1/60 of the IC₅₀ (the drug concentration at which 50% of the cells die) and continuing with subcultures where PTX concentration was increased by 25% every fifteen days. The cells are defined as PTX-resistant when they are able to grow exponentially in the presence of a PTX concentration equal to the IC₅₀ of the sensitive counterpart. The use of the HGC27-R cell line allows *in vitro* simulation of what happens in patients, who after a number of paclitaxel therapy cycles tend to develop chemoresistance, no longer responding to treatment and therefore experiencing disease progression.

The effect of the CC48 compound was tested in parallel on both the HGC27-S and HGC27-R cell lines to assess its action on the main pathways involved in tumor growth, whether used alone or in combination with PTX. Western blotting experiments were performed on the gastric carcinoma cells to assess therein the level of expression of the most important proteins involved in the regulation of the cell proliferation pathway and in the apoptotic pathway, both after treatment with CC48 alone and after treatment with the combination of CC48 and PTX. CC48 has been shown to inhibit the expression of proteins involved in proliferation and to induce the expression of proteins involved in the pro-apoptotic cascade, both in PTX-sensitive and PTX-resistant cells, and its effect has been shown to become even more significant when it is used in combination with PTX.

Flow cytometry experiments were also carried out to confirm these results, in which the action of the CC48 compound was assessed on actively proliferating cells, which express the Ki67 protein at the nuclear level, unlike quiescent cells which are negative for the expression of this marker. In this experiment, administration of CC48 alone was found to exert a non-prominent effect in reducing the percentage of actively proliferating PTX-sensitive cells; however, the effect becomes statistically significant in PTX-resistant cells when the CC48 compound is used in combination with PTX.

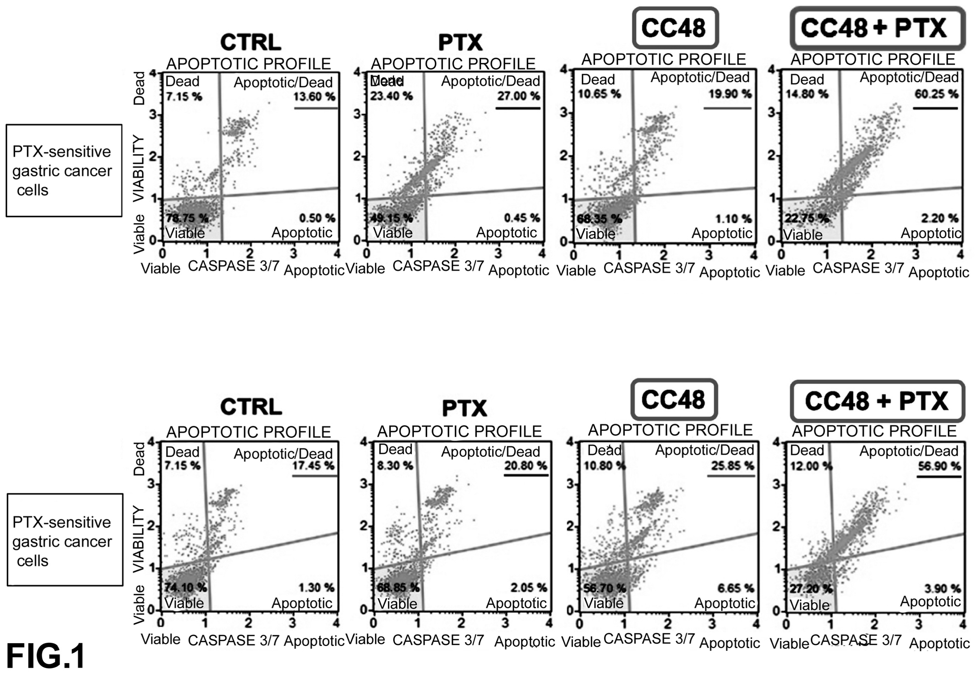

In a second flow cytometry experiment, PTX-sensitive and PTX-resistant cells were labelled with the reagent for caspases 3/7, which are proteins responsible for the apoptotic process, and with the dye 7-ADD, which stains dead cells. Figure 1 shows the results obtained. The graphs in Figure 1 show four quadrants in which the cells have been arranged according to their shape and staining: viable cells in the lower left quadrant, cells in the early stages of the apoptotic process in the lower right quadrant, cells undergoing apoptosis and moving towards cell death in the upper left quadrant, and lastly dead cells in the upper left quadrant.

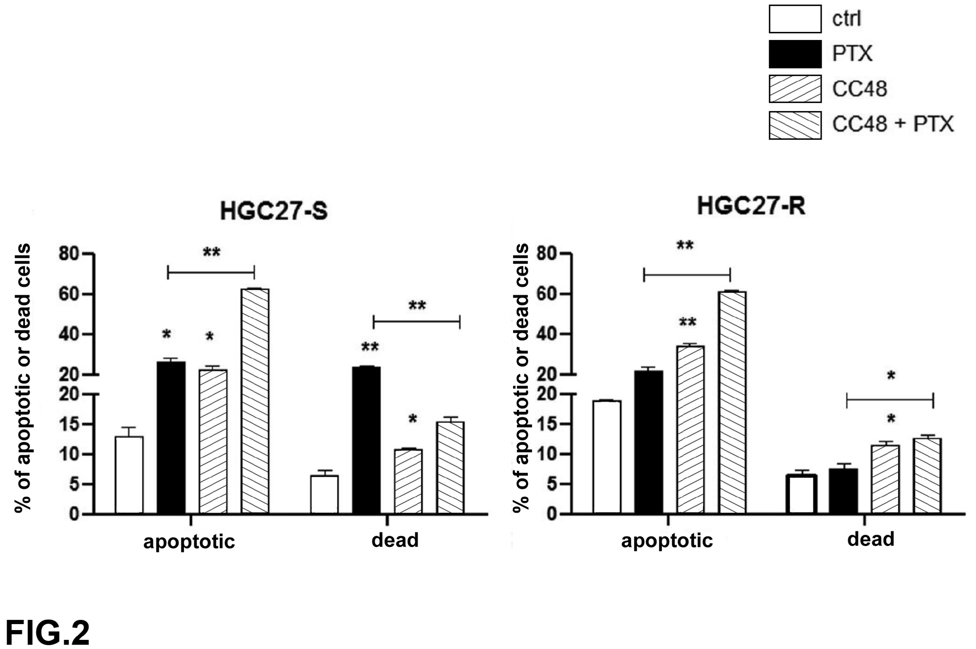

The same results were also plotted in figures 2A and 2B, where Figure 2A shows the results obtained in PTX-sensitive cells, while Figure 2B shows the results obtained in PTX-resistant cells. In sensitive cells, compared to the untreated control, a statistically significant effect of the CC48 compound on apoptosis was observed, comparable to the effect achieved with PTX. In these cells, following combined treatment with CC48 and PTX, a significant increase in the percentage of apoptotic cells was also observed, which was statistically greater than both the effect achieved with PTX alone and the sum of the effects achieved with CC48 and PTX used separately. The data obtained thus show synergy between CC48 and PTX. With regard to the effect on cell death, treatment with CC48 alone was found to be less cytotoxic than treatment with PTX alone. Furthermore, treatment with the combination of CC48 and PTX showed a statistically lower cytotoxicity than treatment with PTX alone.

Even more interesting were the results obtained with PTX-resistant cells, where it was clearly shown that PTX does not exert a significant effect on apoptosis or cell death. In contrast, treatment with CC48 alone in these cells significantly increased the percentage of cells undergoing apoptosis and dead cells. Even more interesting was the effect of the combination of CC48 and PTX, which resulted in a reversal of the cells' resistance to PTX, by exerting a synergistic effect on the percentage of cells undergoing apoptosis but without causing a significant increase in cytotoxicity.

The apoptotic pathway was investigated using a further flow cytometry experiment in which the cells were labelled with Annexin V, a protein that binds specifically to a phospholipid on the outer surface of the cell membrane during apoptosis. The results obtained are comparable to those obtained in the above-described experiment and confirm the synergistic effect of CC48 with PTX, both in PTX-sensitive and PTX-resistant cells.

An experiment was then carried out to assess the effects of the CC48 molecule on cell migration ability. CC48 was shown to inhibit cell migration, i.e., the process underlying tumor metastasization. A cell secretion experiment also demonstrated that the CC48 molecule exerts an inhibitory effect on the secretion of VEGF, a critical molecule for the angiogenic process underlying tumor growth.

Lastly, further *in vitro* experiments demonstrated that treatment with CC48 also leads to a significant increase in the production of ROS (Reactive Oxygen Species) by gastric cancer cells, as well as an increase in proteins involved in autophagy.

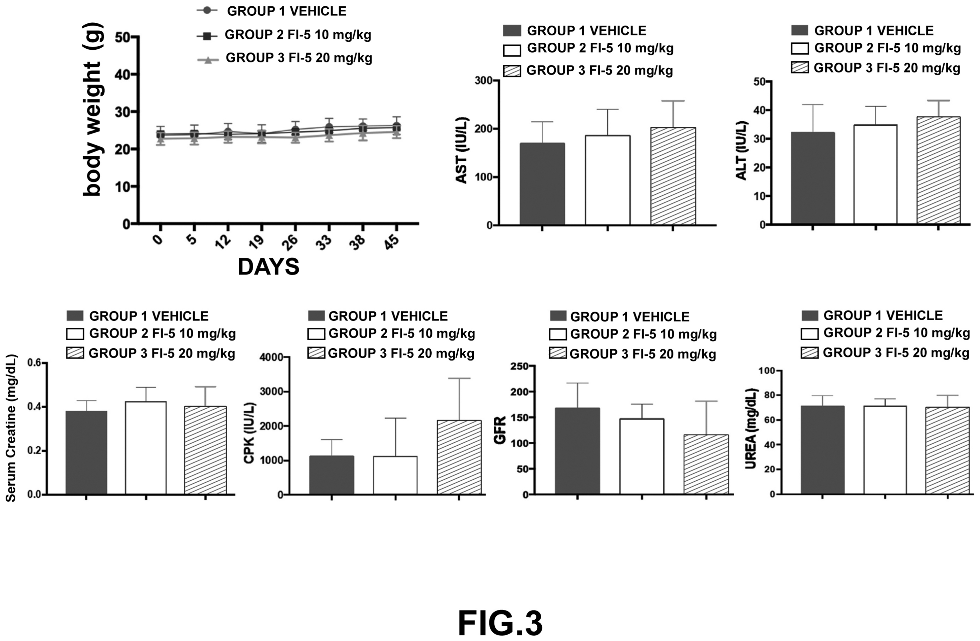

As regards *in vivo* studies, PTX-sensitive gastric carcinoma cells were injected intraperitoneally into 18 nude mice to create mouse models on which to study the effects of the CC48 molecule and its possible side effects. The animals were randomized into 3 experimental groups after Day 5, when tumor engraftment occurred. Group 1 was treated with the vehicle only, while Group 2 and Group 3 were treated with two different doses of CC48, a lower dose (10 mg/kg) and a higher dose (20 mg/kg). The body weight (BW) of each animal was recorded weekly, starting from Day 0 until the end of the study. Tumor growth was measured twice a week. Physical observations, including visual inspection of the general appearance of the mice and their state of health, were carried out daily. No statistically significant differences in body weight were observed between the control group (vehicle) and the two treated groups (GP2 and GP3). Similarly, the biochemical and urinary parameters tested showed no significant alteration in the treated groups. These results are shown in the graphs in Figure 3. No animals showed signs of suffering during the study and no signs of toxicity or organ pathological changes caused by CC48 were found during the necropsy examination.

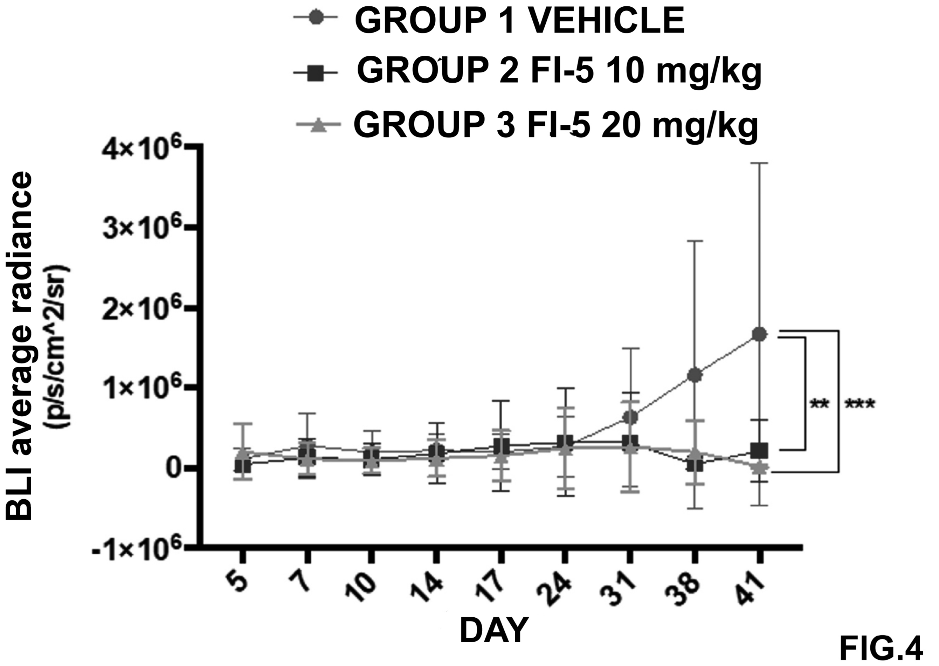

Statistical analysis showed that CC48 has a major antitumor effect. CC48, in fact, slows down the growth of tumor masses either through administration of a low dose of the drug (Group 2, p≤0.05) or through administration of a high dose of the drug (Group 3, p≤0,001). It was therefore concluded that CC48 has a positive effect in reducing tumor growth at both low and high doses. These results are shown in the graph in Figure 4. The data obtained indicate that CC48 has a strong therapeutic potential against gastric cancer cells, as it can significantly reduce tumor masses in mice at both minimal and high doses. Statistical significance indicates that the observed effect is not random, and the reduction in tumor growth can be considered a tangible indication of effectiveness.

In addition, CC48 acts without noticeable toxicity. The lack of toxicity, even at high doses, indicates that CC48 may have a broad therapeutic window, making it an ideal candidate for extended treatment or maintenance therapies.

### EXPERIMENTAL SECTION

### Materials and Methods

### In Vitro Experiments

### Cells and compounds tested

Human gastric carcinoma cell lines HGC27 and AGS were purchased from and authenticated by the American Type Culture Collection (Manassas, Virginia, USA). All cell culture components were purchased from Sigma-Aldrich (Milan, Italy) and Celbio s.r.l. (Milan, Italy). PTX-resistant HGC27 cells (HGC27-R) were obtained by exposure to increasing concentrations of the chemotherapeutic agent, starting with a concentration of 1/60 of the IC₅₀ and continuing with subcultures in which the concentration was increased by 25% every two weeks [Zhang X, Yashiro M, Qiu H, et al (2010) Establishment and characterization of multidrug-resistant gastric cancer cell lines. Anticancer Res 30:915-21]. The cells are considered as resistant when they are able to grow exponentially in the presence of a PTX concentration equal to the IC₅₀ of the sensitive counterpart. HGC27-R cells were grown under the same experimental conditions as their sensitive counterpart with the addition of 9 nM PTX. All experiments comparing resistant and sensitive cell lines were performed at a PTX concentration close to the IC₅₀ of the sensitive cells. PTX was purchased from Teva Italia S.r.l. (Milan, Italy), AM630 was purchased from Tocris S.r.l. (Milan, Italy), while derivatives 1, Fi5 (CC48), Fi9 and ASF151, already published, were synthesized according to literature reports. 2',7'-Dichlorofluorescein diacetate, DCF-DA, was purchased from Sigma-Aldrich (Milan, Italy). Calcein-AM was obtained from Sigma-Aldrich (Milan, Italy).

### Cell cultures

Human gastric carcinoma cell lines HGC27-S/R, AGS, NCl-N87 and KATOIII were cultured in high-glucose RPMI supplemented with 10% fetal bovine serum, 2 mM glutamine, 100 U/mL penicillin, 100 µg/mL streptomycin, in a humidified incubator at 37°C and a 5% CO₂ atmosphere. MDCK-MDR1 cells were a gift from Prof. P. Borst, NKI-AVL Institute, Amsterdam, the Netherlands. MDCK-MDR1 cells were cultured in high-glucose DMEM supplemented with 10% fetal bovine serum, 2 mM glutamine, 100 U/mL penicillin, and 100 mg/mL streptomycin in a humidified incubator at 37°C and a 5% CO₂ atmosphere.

### Gene expression analysis

Total RNA was extracted from GC cells using the Qiagen RNeasy Mini Kit (Qiagen, Hilden, Germany) following the manufacturer's instructions. The samples were reverse transcribed using the iScript Advanced cDNA Synthesis Kit (Bio-Rad Laboratories, California, USA). The cDNA samples were tested by Real-Time-PCR for the expression of CB1R and CB2R. The experiments were performed in triplicate using SsoAdvanced Universal SYBR Green Supermix (Bio-Rad Laboratories, California, USA) on a CFX96 Touch Real-Time PCR detection system (Bio-Rad Laboratories, California, USA) according to the manufacturer's instructions. mRNA expression was normalized to the GAPDH housekeeping gene. PrevalidatedPrimePCR templates for the SYBR Green Assay (Bio-Rad Laboratories, California, USA) were used for the reactions. Relative quantification was performed using the ddCT method.

### FAAH inhibition assay

The assays were performed using black, flat-bottom 96-well NBS plates (COSTAR flat black). Experiments were performed in a total volume of 200 µl, first incubating different concentrations of each potential inhibitor in an appropriate fluorimetric assay buffer (125 mM Tris-HCl, 1 mM Na₂EDTA 2H₂O, pH=9.0) with the enzyme (recombinant human FAAH, Cayman Chemical, Ann Arbor, MI, USA) for 10 minutes at room temperature, while maintaining the plate under orbital shaking. The substrate (7-amino-4-methyl-2H-1-benzopyran-2-one-5Z,8Z,11Z,14Z-eicosatetraen-amide, AMC-AA, final concentration 1µM) was then added and the assay was incubated for 2 h at 37°C in a TECAN infinite M1000Pro plate reader (Tecan, Männedorf, Switzerland) which measured the fluorescence of each well every 30 seconds (λex = 340 nm, λem = 450 nm), determining FAAH activity as Relative Fluorescence Units (RFUs). The inhibitor-free control wells and the inhibitor- and enzyme-free blank wells were used to calculate the percent inhibition of each tested compound. IC₅₀ values were calculated with GraphPad Prism 5.0 (GraphPad Software, La Jolla, CA, USA) and are shown as the mean ± SEM of at least three independent measurements performed in triplicate.

### Cytotoxicity assay

Cell viability was tested by MTT assay at 48 hours and 72 hours [Contino M, Guglielmo S, Riganti C, et al (2020) One molecule two goals: A selective P-glycoprotein modulator increases drug transport across gastro-intestinal barrier and recovers doxorubicin toxicity in multidrug resistant cancer cells. Eur J Med Chem208:112843.https://doi.org/10.1016/j.ejmech.2020.112843]. On Day 1, 5,000 cells/well were seeded in 96-well plates in a volume of 100 µL. On day 2, the compounds to be tested were added at the following concentrations (0.1µM, 1µM, 10µM, 30µM, 50µM, 100µM). In all experiments, the various solvents of the compounds (ethanol, DMSO) were added to each control to assess the possible cytotoxicity of the solvents. After the established time of incubation with the drugs (48h, 72h), MTT (0.5 mg/mL) was added to each well and after 3 hours of incubation at 37°C, the supernatant was removed. Formazan crystals were solubilized with 100 µL of DMSO, and absorbance values at 570 and 630 nm were determined with the PerkinElmer Life Sciences Victor 3 microplate reader. The results are expressed as mean ± SD of 3 independent experiments in triplicate.

### Protein expression analysis

Western blotting analysis was performed as previously described [Carr BI, D'Alessandro R, Refolo MG, et al (2013) Effects of low concentrations of regorafenib and sorafenib on human HCC cell AFP, migration, invasion, and growth in vitro. J Cell Physiol 228:1344-50.https://doi.org/10.1002/jcp.24291] in both HGC27-S/R and AGS cells. Briefly, for each experimental condition the cells were lysed in a cold lysis buffer (50 mM Tris, 10 mM EDTA, 1% v/v Triton-X100) supplemented with the protease/phosphatase inhibitor cocktail (Merck KGaA, Darmstadt, Germany), incubated on ice for 15 minutes and centrifuged at 13,000 × g for 15 minutes at 4°C. Protein extracts were quantified with the Micro BCA^{™} Protein Assay Kit (Thermo Fisher Scientific Inc., MA USA), and 40 µg of total protein extract were loaded onto SDS-PAGE and immunoblotted with the following antibodies: CB1R and CB2R (1:500, rabbit polyclonal, Abcam, Cambridge, UK), P-TSC2 (Ser939) and TSC2 (1:1000 Cell Signaling, Beverly, MA, USA), P-PI3K p85 (Tyr458)/p55(Tyrl99) and PI3K (1:1000 Cell Signaling, Beverly, MA, USA), P-P70 (Thr389) and P70 (1:1000 Cell Signaling, Beverly, MA, USA), P-Akt (Ser473 thr308), and Akt (1:1000 Cell Signaling, Beverly, MA, USA), P-S6 (Ser235/236) and S6 (1:1000 Cell Signaling, Beverly, MA, USA), 4EBP1 (1:1000 Cell Signaling, Beverly, MA, USA), P-erk (Thr202/Tyr204) and erk (1:1000 Cell Signaling, Beverly, MA, USA), PPRγ (1:1000 Cell Signaling, Beverly, MA, USA), \Phospho-SAPK/JNK (Thr183/Tyr185) and JNK2 (1:1000 Cell Signaling, Beverly, MA, USA), Phospho-c-Jun (Ser63) and c-Jun (1:1000 Cell Signaling, Beverly, MA, USA), caspase 3/7 (1:1000 Cell Signaling, Beverly, MA, USA), phospho-β-catenin (Ser675) and β-catenin (1:1000 Cell Signaling, Beverly, MA, USA), vimentin (1:1000 Cell Signaling, Beverly, MA, USA), P-cofilin (Ser3) and cofilin (1:1000 Cell Signaling, Beverly, MA, USA), actin (1:1000 Cell Signaling, Beverly, MA, USA). Subsequently, the membranes were incubated with the corresponding secondary antibodies conjugated with horseradish peroxidase (HRP) (Bio-Rad, Hercules, CA, USA). An enhanced chemiluminescence kit (Bio-Rad, Hercules, CA, USA) was used. A Chemidoc XRS+ and the Bio-rad software (Bio-Rad, Hercules, CA, USA) were used to observe and analyze the chemiluminescence signals of the proteins. Total protein expression was quantified with the ImageJ open source software.

### Ki67 cell proliferation assay

HGC27-S/R cells were treated with 4 nM PTX and 6 µM AM630, 10µM Fi5 (CC48), 10 µM Fi9, 10 µM ASF151 and 10 µM Reference 1, administered alone or in combination for 48 h. Following the specified drug treatments, the cells were analyzed with the Muse Ki67 cell proliferation kit, which identifies actively proliferating cells based on the expression of Ki67, a nuclear protein present in the active phases of the cell cycle (phases G1, S, G2 and M) and absent in the resting GO phase. The Muse Ki67 Proliferation Assay was used with the Guava Muse Cell Analyzer according to the manufacturer's instructions (Luminex Corporation, Austin, USA). Briefly, the cells were stained after the fixation and permeabilization procedures using antibodies conjugated with fluorochrome Hu Ki67 or control Hu IgG1 to distinguish between Ki67+ or Ki67- cells, respectively. The software provided the percentage of Ki67(+) and Ki67(-) cells.

### P-gp interaction assay

This experiment was performed as described by Contino et al. with small modifications [Contino M, Guglielmo S, Riganti C, et al (2020) One molecule two goals: A selective P-glycoprotein modulator increases drug transport across gastro-intestinal barrier and recovers doxorubicin toxicity in multidrug resistant cancer cells. Eur J Med Chem208:112843.https://doi.org/10.1016/j.ejmech.2020.112843]. MDCK-MDR1 cells, constitutively over-expressing P-gp, (30,000 cells per well) were seeded in CulturePlate96/black well plates with 100 µL of medium and allowed to become confluent overnight. Each tested compound, solubilized in the culture medium, was added to the cell monolayers, reaching the final tested concentrations (0.1 to 100 µM). The 96-well plate was incubated at 37°C for 30 minutes. 100 µL of pro-fluorescent probe Calcein-AM (2.5 µM in phosphate buffered saline, PBS) were added to each well and the plate was incubated for 30 minutes. Each well was washed 3 times with cold PBS. Saline buffer was added to each well and the plate was read with Victor3 (PerkinElmer) at excitation and emission wavelengths of 485 nm and 535 nm, respectively. Under these experimental conditions, cell accumulation of calcein was assessed in the absence and presence of the tested compounds, and the baseline fluorescence level was estimated compared to untreated cells. In the treated wells, the increase in fluorescence was measured compared to the baseline level. The EC50 values were determined by adjusting the fluorescence increase percentage by log [dose].

### ROS determination assay

The following procedure was adapted for ROS analysis. Briefly, 5 x 10⁵ cells were seeded and incubated for 48 hours in the presence and absence of the reference compounds for CB2R, agonist 1 (Compound 28 in the paper Stern et al. JMC 2006doi: 10.1021/jm050467q) and the antagonist AM630 (doi: 10.1016/0024-3205(95)00175-6), and the CB2R agonists Fi5 (CC48), Fi9 and ASF151. After a 48h incubation, all samples were incubated for 30 minutes at 37°C in the dark with 50 µM 2',7'-dichlorofluorescein diacetate, DCF-DA.

The cells were then washed, detached with trypsin/EDTA solution, centrifuged at 12,000 g for 10 minutes and resuspended in 1 mL of PBS. At least 10,000 total events were captured using an Attune Nxt acoustic focusing cytometer (Life Technologies Corporation) equipped with a blue laser (488 nm). The analysis was performed, and the data was displayed using Attune^{™} NxT v2.6 software. The percentage of fluorescent cells and the median fluorescence were measured using a single-parameter histogram based on DCF-DA fluorescence.

### Autophagy assays

Cells were rinsed with cold lysis buffer (50 mM Tris, 10 mM EDTA, 1% v/v Triton-X100) supplemented with protease inhibitor cocktail III (80 µM aprotinin, 5 mM bestatin, 1.5 mM leupeptin, 1 mM pepstatin; Sigma-Merck St. Louis, MO), 2 mM phenylmethyl sulfonyl fluoride, and 1 mM Na₃VO₄, then sonicated and centrifuged at 13,000 g for 10 minutes at 4°C. 20 µg of protein extracts were subjected to SDS-PAGE and tested with the following antibodies: anti-ATG5 (#ab108327, diluted 1/1000), anti-ATG7 (#ab52472, diluted 1/5000), anti-ATG12 (#ab303488, diluted 1/1000), anti-Beclin (#ab210498, diluted 1/1000), anti-p62 (#ab109012, diluted 1/10000), anti-LC3 (#ab192890, diluted 1/2000) (all from Abcam, Cambridge, UK) or anti-β-tubulin antibody (#sc-5274, diluted 1/1000; Santa Cruz Biotechnology Inc., Santa Cruz, CA, USA), followed by incubation with a peroxidase-conjugated secondary antibody (Bio-Rad Laboratories, Hercules, CA). After washing in 0.1% v/v Tris-buffered saline (TBS)-Tween, proteins were detected by enhanced chemiluminescence (Bio-Rad Laboratories).

### Apoptosis assays

HGC27-S/R and AGS cells were treated with 1-6 µM AM630, 1-10 µM Fi5 (CC48), 1-10 µM Fi9, 1-10 µM ASF151 and 1-10 µM Compound 1 administered for 48 hours. Following the specified drug treatments, the cells were processed with the Muse Annexin V/Dead Cell Assay Kit (Luminex Corporation, Austin, USA) for quantitative analysis of viable, early/late apoptotic and dead cells on a Muse Cell Analyzer. Briefly, the test uses Annexin V to detect phosphatidyl serine on the outer membrane of apoptotic cells. The fluorescent signal emitted by the dye-conjugated antibodies was detected using flow cytometry technology (Muse Cell Analyzer, Luminex Corporation, Austin, USA). The cell death marker 7-amino-actinomycin D (7-AAD) was also used. In addition, HGC27-S/R cells were treated with 4 nM PTX alone or in combination with 6 µM AM630, 10 µM Fi5 (CC48), Fi9, ASF151 and 1 for the assessment of the apoptotic state based on Caspase3/7 activation, and for cell death analysis by determining cell plasma membrane permeabilization. After 48 hours of treatment, the cells were treated according to the instructions for use and analyzed with the Muse Caspase-3/7 kit (Millipore). The test provides the relative percentage of viable, early/late apoptotic or dead cells.

### Migration assay

HGC27-S/R and AGS cells were grown to confluence. A scratch was done with a pipette tip. After washing with the medium to remove the detached cells, a low-serum medium (1% FBS) with 1-6 µM AM630, 1-10 µM Fi5 (CC48), 1-10 µM Fi9, 1-10 µM ASF151, and 1-10 µM compound 1 was added. Photographs of each well were taken immediately (T0) and after various times at T1 (9 h), T2 (24 h) and T3 (48 h), using a Leica DMRXA camera (Leica Microsystems, Milan, Italy). The images were analyzed with the ImageJ open source software. The distance travelled by the cells across the scratched area was determined by measuring the width of the scratch at T1, T2, and T3 and subtracting it from the wound width at baseline (T0). The relative migration rate was calculated by setting the percentage of control cell migration at time T2 equal to 1 and comparing the percentage of cell migration after each drug treatment with this value. The results represent three independent experiments.

### VEGFA measurement in cell culture media

The amount of VEGFA secreted into the culture medium by treated HGC27-S/R and AGS cells was measured using a highly sensitive Quantikine ELISA (Enzyme-Linked Immunosorbent Assay) kit (R&D Systems, Minneapolis, MN, USA) according to the manufacturer's instructions. The measured values were normalized to the number of cells.

### Statistical analysis

GraphPad Prism 5.0 software (La Jolla, CA, USA) was used to assess differences between two unpaired groups using the Mann-Whitney non-parametric test. P<0.05 was considered statistically significant. All experiments were performed in triplicate and repeated three times. Data were presented as mean ± standard deviation (SD).

### In Vivo Experiments

18 female nude mice were injected intraperitoneally with the NCI-N87_LUC cell line, at a concentration of 2x10⁶/100 *µ*l cells.

The experimental groups and the treatments are described in the table below.

| **Experimental group (GP)** | **Sex** | **No. animals** | **Animal IDs From - To** | **Cell line** | **Treatment** | **Dose** | **Route** | **Regime** |
|---|---|---|---|---|---|---|---|---|
| **1** | F | 6 | 1-6 | NCI-N87_LUC | VEHICLE | - | IP | Every other day |
| **2** | F | 6 | 7 - 12 | NCI-N87_LUC | Fi5 (CC48) | 10 mg/kg | IP | Every other day |
| **3** | F | 6 | 13 - 18 | NCI-N87_LUC | Fi5 (CC48) | 20 mg/kg | IP | Every other day |

TPS administrations were performed by intraperitoneal (IP) injection as shown in Table 1, starting from the 5th day of the study, considering an administration volume of 10 ml/kg.

All animals were fed ad libitum with complete mouse feed and water was supplied ad libitum.

The body weight of each animal was recorded weekly, starting from Day 0 until the end of the study.

Tumor growth was assessed weekly using the *in vivo* IVIS Spectrum technology system (PerkinElmer) by intraperitoneal injection of D-Luciferin potassium salt (100 µL/10 gr, PerkinElmer).

Physical observations, including visual inspection of the general appearance of the mice (e.g., hair and activity/reactivity levels) and their state of health, were carried out daily.

On Day 45, the animals were housed individually in metabolic cages for 24 hours, with free access to food and water, in order to collect the urine. Immediately prior to placement in the metabolic cage, the animals were subjected to a water load (10 ml/kg orally) to allow normalization of the urine volume. Urine was used to perform urinalysis.

Moreover, at the end of the study period, the animals underwent blood sampling from the submandibular vein, after isoflurane anesthesia and before sacrifice. The blood was collected in Eppendorf tubes and then centrifuged at approximately 8,000 rpm for 15 minutes at 4°C for serum collection.

The animals were sacrificed with CO2 and subjected to tumor sampling. Tumor masses, when possible and present, were divided into two halves, one frozen at -80° and the other preserved in 10% buffered formalin.

### Results and discussion

On the fifth day, following IVIS acquisition, the 18 female CD1 nude mice were randomized and divided into three groups (3 mice per cage). Each animal received 2x10⁶/100*µ*l NCI-N87_LUC cells intraperitoneally and was weighed once a week. No animals showed signs of suffering or discomfort during the study and no signs of toxicity or organ pathological changes caused by the drug Fi5 (CC48) were found during the necropsy examination.

Tumour masses were measured using the PerkinElmer IVIS spectrum twice a week and statistical analysis showed that Fi5 (CC48) has a major antitumor effect on NCI-N87_LUC; in fact, it slows down the growth of tumor masses either through administration of a low dose of the drug (GP2, p≤0.05) or through administration of a high dose of the drug (GP3, p≤0.001).

During the study, the test drug (CC48) was found to exert a positive effect in reducing tumor growth at both low and high doses.

No statistically significant differences in body weight were observed between the control group (vehicle) and the two treated groups (GP2 and GP3), suggesting that the drug did not adversely affect the growth or energy balance of the animals.

Similarly, the biochemical and urinary parameters tested showed no significant alterations in the treated groups (GP2 and GP3), when compared to the control group. These results indicate that the drug had no adverse or toxic effects on the main physiological indicators monitored during the treatment period.

## Claims

1. A compound of formula (I): 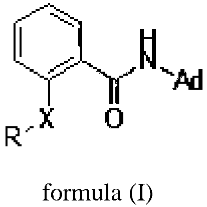 wherein:
X is selected from NH and O,
Ad is adamantyl,
R is selected from the group consisting of linear C1-C5 alkyl, phenyl, benzyl, and methyl cyclohexyl,
for use in the therapeutic treatment of gastric carcinoma.

2. The compound for use according to claim 1, wherein the compound is selected from the group consisting of:

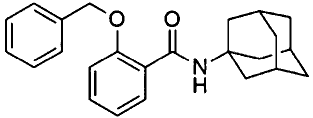

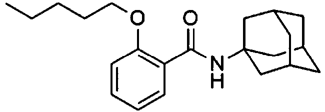

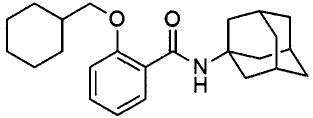

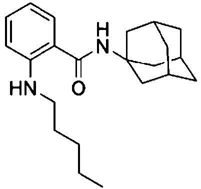

3. The compound for use according to claim 1, wherein the compound is:

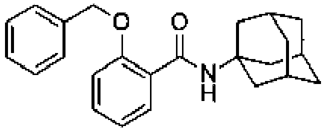

4. The compound for use according to any of claims 1 to 3, wherein the gastric carcinoma is advanced gastric carcinoma.

5. The compound for use according to any of claims 1 to 4, wherein the therapeutic treatment comprises the administration of one or more chemotherapeutic agents.

6. The compound for use according to claim 5, wherein the therapeutic treatment comprises the administration of paclitaxel optionally in combination with Ramucirumab.

7. The compound for use according to any of claims 1 to 6, formulated for oral or intravenous administration.

8. A combined preparation comprising:
(i) a compound of formula (I): 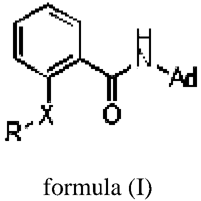 wherein:
X is selected from NH and O,
Ad is adamantyl,
R is selected from the group consisting of linear C1-C5 alkyl, phenyl, benzyl, and methyl cyclohexyl, and
(ii) one or more chemotherapeutic agents, for simultaneous, separate or sequential use in the treatment of gastric carcinoma.

9. The combined preparation for use according to claim 8, wherein the compound is selected from the group consisting of:

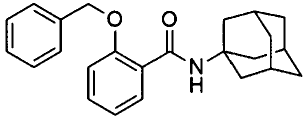

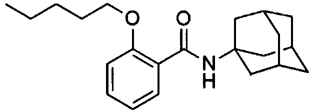

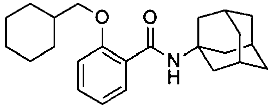

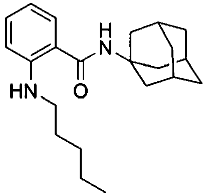

10. The combined preparation for use according to claim 9, wherein the compound is:

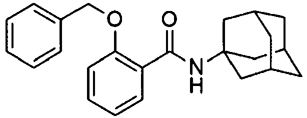

11. The combined preparation for use according to any of claims 8 to 10, wherein the gastric carcinoma is advanced gastric carcinoma.

12. The combined preparation for use according to any of claims 8 to 11, wherein the therapeutic treatment comprises the administration of one or more chemotherapeutic agents.

13. The combined preparation for use according to claim 12, wherein the therapeutic treatment comprises the administration of paclitaxel optionally in combination with Ramucirumab.

14. The combined preparation for use according to any of claims 8 to 13, wherein the compound of formula (I) is formulated for oral or intravenous administration.

15. A pharmaceutical composition comprising a compound of formula (I) as defined in any of claims 1 to 3 and one or more pharmaceutically acceptable carriers or excipients, optionally in combination with one or more chemotherapeutic agents, for use in the therapeutic treatment of gastric carcinoma.

16. The pharmaceutical composition for use according to claim 15, comprising the therapeutic agent paclitaxel optionally in combination with Ramucirumab.
